# EUROPEAN PATENT APPLICATION

(11) **EP 4 094 702 A2**
(43) Date of publication of application: **30.11.2022**
(21) Application number: 22171402.5
(22) Date of filing: 03.05.2022
(51) Int. Cl.: A61B 18/14, A61B 18/12

(54) **ENERGY ACTIVATION SWITCH FOR VESSEL SEALER**

(30) Priority: 03.05.2021 US 202163183088 P; 08.04.2022 US 202217715986
(71) Applicant: Covidien LP, Mansfield, MA 02048 (US)
(72) Inventor: LYONS, Michael, B, Boulder, CO Colorado 80301 (US); CROFT, Richard, L, Boulder, CO Colorado 80301 (US); VAN TOL, David, J, Boulder, CO Colorado 80301 (US)
(74) Representative: Maschio, Antonio

(57) **Abstract**

An endoscopic surgical forceps includes a housing having a shaft extending therefrom with an end effector operably coupled thereto. The end effector includes first and second jaw members movable relative to one another between open and closed positions. A first handle is pivotably coupled to the housing and is movable relative to a second handle disposed on the housing, the first handle configured to actuate the jaw members between the open and closed positions upon movement thereof relative to the second handle. A trigger is operably coupled to the housing and is configured to deploy a knife between the first and second jaw members upon actuation thereof. An energy activation switch is coupled to an electrosurgical energy source such that activation thereof supplies energy to the first and second jaw members, the energy activation switch is disposed on the trigger.

## Description

### CROSS-REFERENCE TO RELATED APPLICATION

This application claims the benefit of U.S. Provisional Patent Application No. 63/183,088, filed May 3, 2021, the entire disclosure of which is incorporated by reference herein.

### BACKGROUND

### 1. Technical Field

The present disclosure relates generally to the field of surgical instruments. In particular, the disclosure relates to an endoscopic electrosurgical forceps that includes an ergonomically-located energy activation switch for facilitating energy delivery.

### 2. Background of Related Art

Instruments such as electrosurgical forceps are commonly used in open and endoscopic surgical procedures to coagulate, cauterize and seal tissue. Such forceps typically include a pair of jaw members that can be controlled by a surgeon to grasp targeted tissue, such as, e.g., a blood vessel. The jaw members may be approximated to apply a mechanical clamping force to the tissue, and are associated with at least one electrode to permit the delivery of electrosurgical energy to the tissue. The combination of the mechanical clamping force and the electrosurgical energy has been demonstrated to join adjacent layers of tissue captured between the jaw members. When the adjacent layers of tissue include the walls of a blood vessel, sealing the tissue may result in hemostasis, which may facilitate the transection of the sealed tissue. A detailed discussion of the use of an electrosurgical forceps may be found in U.S. Patent No 7,255,697 to Dycus et al.

A bipolar electrosurgical forceps typically includes opposed electrodes disposed on clamping faces of the jaw members. The electrodes are charged to opposite electrical potentials such that an electrosurgical current may be selectively transferred through tissue grasped between the electrodes. To effect a proper seal, particularly in relatively large vessels, two predominant mechanical parameters must be accurately controlled; the pressure applied to the vessel, and the gap distance established between the electrodes.

Both the pressure and gap distance influence the effectiveness of the resultant tissue seal. If an adequate gap distance is not maintained, there is a possibility that the opposed electrodes will contact one another, which may cause a short circuit and prevent energy from being transferred through the tissue. Also, if too low a force is applied the tissue may have a tendency to move before an adequate seal can be generated. The thickness of a typical effective tissue seal is optimally between about 0.001 and about 0.006 inches. Below this range, the seal may shred or tear and above this range the vessel walls may not be effectively joined. Closure pressures for sealing tissue structures fall within the range of about 3kg/cm² to about 16 kg/cm².

Typically, manufactures have developed vessel sealing forceps which include an activation handle utilized for compressing tissue between the jaw members within the above pressure range upon actuation thereof. Once compressed, the handle may be held in place or locked in place while the user activates an energy activation switch disposed on the housing with his/her thumb or forefinger, e.g., thumb if located on a proximal side thereof, or forefinger if located along the side thereof.

In order to provide a more progressive approach to sealing vessels, manufacturers have developed so-called in-line activation vessel sealing devices wherein actuation of a handle through the handle's range of motion initially actuates the jaw members to grasp and compress tissue under the appropriate closure pressures for sealing vessels and continued actuation of the handle subsequently activates an energy activation switch for supplying energy to the jaw members for sealing the tissue disposed therebetween.

In some instances it may be desirable to include a more ergonomically-friendly location for the energy activation switch.

### SUMMARY

The present disclosure relates generally to the field of surgical instruments. In particular, the disclosure relates to an endoscopic electrosurgical forceps that includes a system and method for springing open jaw members.

As is traditional, the term "distal" refers herein to an end of the apparatus that is farther from an operator, and the term "proximal" refers herein to the end of the electrosurgical forceps that is closer to the operator.

The present disclosure relates to an endoscopic surgical forceps and includes a housing having an elongated shaft including a distal portion extending therefrom, a proximal portion coupled to the housing, and a longitudinal axis defined therethrough. An end effector assembly is operably coupled to a distal end of the elongated shaft, the end effector including first and second jaw members. One or both of the jaw members is movable relative to the other jaw member between an open position wherein the jaw members are spaced relative to each other and a closed position wherein the jaw members cooperate to grasp tissue therebetween. A first handle is pivotably (or otherwise operably) coupled to the housing and is movable relative to a second handle disposed on the housing. The first handle is operably coupled to the jaw member(s) and is configured to actuate the jaw member(s) between the open and closed positions upon movement thereof relative to the second handle.

A trigger is operably coupled to the housing and is configured to deploy a knife between the first and second jaw members upon actuation thereof. An energy activation switch is adapted to couple to an electrosurgical energy source such that activation thereof supplies energy to the jaw members, the energy activation switch is disposed on the trigger, within the housing or handle.

In aspects according to the present disclosure, actuation of the trigger requires a first force and activation of the energy activation switch requires a second force, the first force being greater than the second force. In other aspects according to the present disclosure, the energy activation switch is adapted to connect to an electrosurgical energy source capable of supplying multiple energy modalities to the jaw members upon activation thereof and wherein the energy modality of the energy activation switch is dependent on the position of the first handle relative to the second handle.

In aspects according to the present disclosure, when the first handle is disposed in an open position, the energy activation switch, when activated, provides monopolar energy from the electrosurgical energy source to the one or both jaw members. In other aspects according to the present disclosure, when the first handle is disposed in a partially closed position, the energy activation switch, when activated, provides bipolar energy from the electrosurgical energy source to the first and second jaw members. In still other aspects according to the present disclosure, when the first handle is disposed in a fully closed position and is generating a closure pressure within the range of about 3kg/cm² to about 16 kg/cm² between the first and second jaw members, the energy activation switch, when activated, provides energy from the electrosurgical energy source according to an algorithm, e.g., Ligasure^{™} algorithm, for sealing tissue between the first and second jaw members.

In aspects according to the present disclosure, the forceps further includes an auxiliary energy activation switch disposed on the second handle, the auxiliary energy activation switch, upon activation, also configured to provide energy from the electrosurgical energy source according to an algorithm, e.g., Ligasure^{™} algorithm, for sealing tissue between the first and second jaw members. In other aspects according to the present disclosure, the auxiliary energy activation switch is activatable by the first handle moving relative to the second handle from an open position to a fully closed position wherein the first handle generates a closure pressure within the range of about 3kg/cm² to about 16 kg/cm² between the first and second jaw members. In still other aspects according to the present disclosure, the energy activation switch, when activated, provides monopolar or bipolar energy to the first and second jaw members depending upon the position of the first handle relative to the second handle.

The present disclosure also relates to an endoscopic surgical forceps that includes a housing having an elongated shaft with a distal portion extending therefrom, a proximal portion coupled to the housing, and a longitudinal axis defined therethrough. An end effector assembly is operably coupled to a distal end of the elongated shaft, the end effector including first and second jaw members. One or both of the first and second jaw members is movable relative to the other jaw member between an open position wherein one or both jaw member(s) is spaced relative to the other jaw member and a closed position wherein the first and second jaw members cooperate to grasp tissue therebetween.

A trigger is operably coupled to the housing and is configured to deploy a knife between the first and second jaw members upon actuation thereof. An energy activation switch is adapted to couple to an electrosurgical energy source such that activation thereof supplies energy to one or both of the first and second jaw members, the energy activation switch is disposed on the trigger.

In aspects according to the present disclosure, the energy activation switch is adapted to connect to an electrosurgical energy source capable of supplying multiple energy modalities to the first and second jaw members upon activation thereof and wherein the energy modality of the energy activation switch is dependent the actuation direction of the energy activation switch relative to the longitudinal axis.

The present disclosure also relates to an endoscopic surgical forceps that includes a housing having an elongated shaft with a distal portion extending therefrom, a proximal portion coupled to the housing, and a longitudinal axis defined therethrough. An end effector assembly is operably coupled to a distal end of the elongated shaft, the end effector including first and second jaw members. One or both of the first and second jaw members is movable relative to the other jaw member between an open position wherein one or both jaw member(s) is spaced relative to the other jaw member and a closed position wherein the first and second jaw members cooperate to grasp tissue therebetween.

A trigger is operably coupled to the housing and is configured to deploy a knife between the first and second jaw members upon actuation thereof. An energy activation switch is adapted to couple to an electrosurgical energy source such that activation thereof supplies energy to one or both of the first and second jaw members, the energy activation switch is disposed on the trigger. A toggle is disposed on the housing and configured to change an energy modality of the energy activation switch between two or more energy modalities.

In aspects according to the present disclosure, the toggle is moveable between a first position enabling the supply monopolar energy from the electrosurgical energy source to one or both of the first and second jaw members upon activation the energy activation switch and a second position enabling the supply of bipolar energy from the electrosurgical energy source to the first and second jaw members upon activation of the energy activation switch.

In aspects according to the present disclosure, the forceps further includes an auxiliary energy activation switch disposed on the second handle, the auxiliary energy activation switch, upon activation, also configured to provide energy from the electrosurgical energy source according to an algorithm e.g., Ligasure^{™} algorithm, for sealing tissue between the first and second jaw members. In other aspects according to the present disclosure, the auxiliary energy activation switch is activatable by the first handle moving relative to the second handle from an open position to a fully closed position wherein the first handle generates a closure pressure within the range of about 3kg/cm² to about 16 kg/cm² between first and second jaw members. In still other aspects according to the present disclosure, the energy activation switch, when activated, provides monopolar or bipolar energy to the first and second jaw members depending upon the position of the first handle relative to the second handle.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1 is a perspective view of a prior art electrosurgical forceps including jaw members at a distal end thereof and an energy activation switch disposed on a housing at a proximal end thereof;
FIG. 2A is a perspective view of another prior art electrosurgical forceps including an energy activation switch disposed in-line with an actuation path of a handle of the electrosurgical forceps;
FIG. 2B is an enlarged, perspective view of an end effector assembly of the embodiment shown in FIG. 2A;
FIG. 3A is a side view of one embodiment of an electrosurgical forceps including an activation switch disposed on a trigger thereof;
FIG. 3B is a side view of another embodiment of the electrosurgical forceps including a dual activation switch disposed both on the trigger and disposed in-line with an actuation path of the handle;
FIG. 3C is a side view of another embodiment of the electrosurgical forceps including an activation switch disposed on a trigger thereof and including a switch selector for allowing activation while the jaw members disposed in an open configuration for dissecting tissue; and
FIG. 3D is a side view of another embodiment of the electrosurgical forceps including a dual activation switch disposed both on the trigger and disposed in-line with an actuation path of the handle and including a switch selector for allowing activation while the jaw members disposed in an open configuration for dissecting tissue.

### DETAILED DESCRIPTION

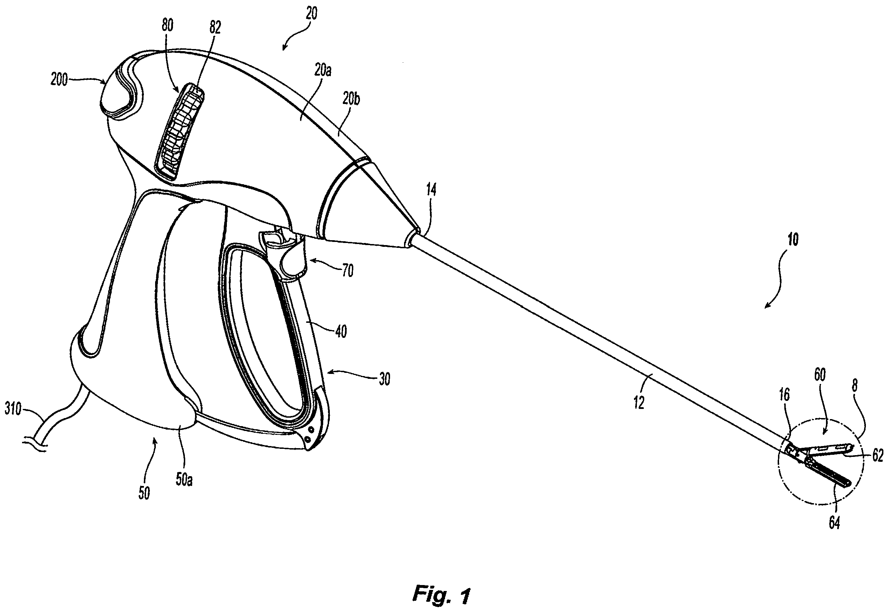

Turning initially to FIG. 1, one embodiment of a prior art endoscopic bipolar forceps 10 is shown for use with various surgical procedures and generally includes a housing 20, a handle assembly 30, a rotating assembly 80, a trigger assembly 70 and an end effector assembly 60 which mutually cooperate to grasp, seal and divide tubular vessels and vascular tissue. For the purposes herein, the forceps 10 is described in terms of an endoscopic instrument, however, it is contemplated that an open version of the forceps may also include the same or similar operating components and features as described below.

Forceps 10 includes a shaft 12 which has a distal end 16 dimensioned to mechanically engage the end effector assembly 60 and a proximal end 14 which mechanically engages the housing 20. The proximal end 14 of shaft 12 is received within the housing 20. In the drawings and in the descriptions which follow, the term "proximal", as is traditional, will refer to the end of the forceps 10 which is closer to the user, while the term "distal" will refer to the end which is further from the user.

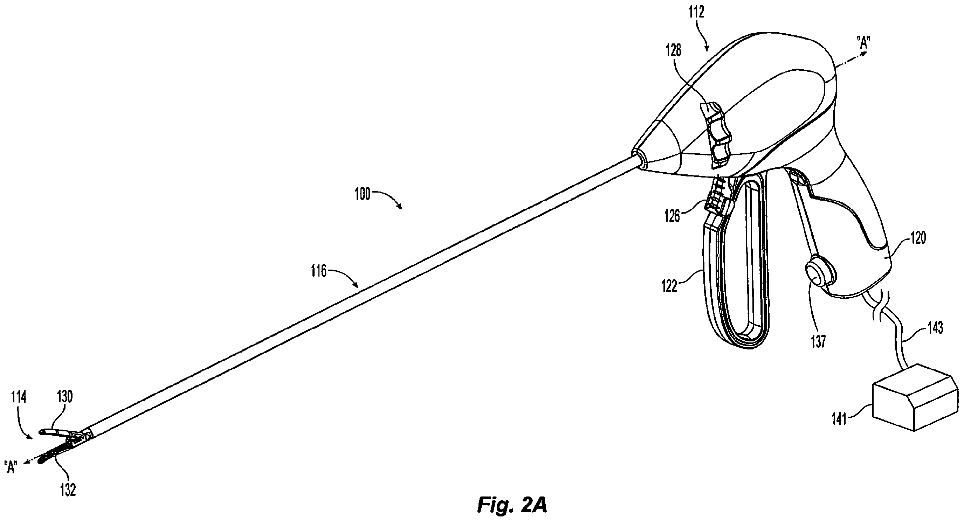

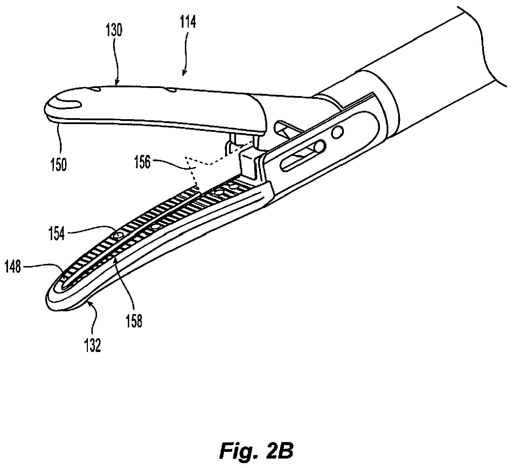

Forceps 10 also includes an electrosurgical cable 310 which connects the forceps 10 to a source of electrosurgical energy, e.g., a generator 141 (FIG. 2). Handle assembly 30 includes a fixed handle 50 and a movable handle 40. Fixed handle 50 is integrally associated with housing 20 and handle 40 is movable relative to fixed handle 50. Actuation of handle 40 relative to handle 50 moves a first jaw member 62 of the end effector assembly 60 relative to a second jaw member 64 of the end effector assembly 60. Rotating assembly 80 is integrally associated with the housing 20 and includes a wheel 82 that is rotatable in either direction to correspondingly rotate the end effector 100. A switch 200 is disposed on a proximal end of the housing 20 and is selectively actuatable by a user's thumb to control the flow of electrosurgical energy from the generator 141 to the end effector assembly 100. A trigger 70 is disposed on the housing 20 and is selectively actuatable by the user's forefinger to advance a knife 156 or an electrosurgical cutting element (not shown) to cut the tissue disposed between the jaw members 62, 64 after the tissue is successfully sealed.

In use, a user manipulates the jaw members 62, 64 about tissue and actuates the handle 40 relative to handle 50 to compress the jaw members 62, 64 about the tissue under the appropriate pressure to seal vessels, e.g., within the range of about 3kg/cm² to about 16 kg/cm². Switch 200 is then actuated by the user's thumb to energize the jaw members 62, 64 to seal tissue therebetween. Once sealed, the user actuates the trigger 70 to cut the tissue between the jaw members 62, 64. As mentioned above, one or more safety features (not shown) may additionally be employed to ensure the jaw members 62, 64 are disposed in the closed or approximated position prior to activation of switch 200, e.g., sensors, internal switches, etc.

As can be appreciated, the user must reorient his/her thumb or finger to activate the switch 200 which may prove to be cumbersome or ergonomically awkward.

Referring initially to FIG. 2A, another embodiment of a prior art electrosurgical forceps 100 generally includes a housing 112 that supports various actuators thereon for remotely controlling an end effector 114 through an elongated shaft 116. To mechanically control the end effector 114, the housing 112 supports a stationary handle 120, a movable handle 122, a trigger 126 and a rotation knob 128. The movable handle 122 is operable to move the end effector 114 between an open configuration wherein a pair of opposed jaw members 130, 132 are disposed in spaced relation relative to one another, and a closed or clamping configuration wherein the jaw members 130, 132 are closer together. Approximation of the movable handle 122 with the stationary handle 120 serves to move the end effector 114 to the closed configuration and separation of the movable handle 122 from the stationary handle 120 serves to move the end effector 114 to the open configuration.

The trigger 126 is operable to extend and retract a knife blade 156 through the end effector 114 when the end effector 114 is in the closed configuration. The rotation knob 128 serves to rotate the elongated shaft 116 and the end effector 114 about a longitudinal axis A-A extending through the forceps 100.

To electrically control the end effector 114, the stationary handle 120 supports a depressible switch 137 thereon which is operable by the user to initiate and terminate the delivery of electrosurgical energy to the end effector 114. More specifically, the depressible switch 137 is mechanically coupled to the stationary handle 120 and is engageable upon proximal movement of the moveable handle 122 to an actuated or proximal position. The switch 137 is in electrical communication with generator 141 or a battery (not shown) supported within the housing 112. The generator 141 may include devices such as the LIGASURE^{®} Vessel Sealing Generator and the Force Triad^{®} Generator sold by Covidien. A cable 143 extends between the housing 112 and the generator 141 and includes a connector (not shown) thereon such that the forceps 100 may be selectively coupled and decoupled electrically from the generator 141.

Referring now to FIGS. 2B, the end effector 114 may be moved from the open configuration wherein tissue is received between the jaw members 130, 132, and the closed configuration, wherein the tissue is clamped and sealed. Jaw members 130, 132 are electrically coupled to cable 143, and thus to the generator 141 (e.g., via a respective wire extending through the elongated shaft 116) to provide an electrical pathway to a pair of electrically conductive, tissue-engaging sealing plates 148, 150 disposed on the jaw members 132, 130, respectively. The sealing plate 148 of jaw member 132 opposes the sealing plate 150 of jaw member 130, and, in some embodiments, the sealing plates 148 and 150 are electrically coupled to opposite terminals, e.g., positive or active (+) and negative or return (-) terminals associated with the generator 141. Thus, bipolar energy may be provided through the sealing plates 148 and 150.

A separation or gap distance may be maintained between the sealing plates 148, 150 by an array of stop members 154 (FIG. 2B) disposed on or adjacent the sealing plates 148, 150. The stop members 154 contact opposing surfaces on the opposing jaw member 130, 132 and prohibit further approximation of the sealing plates 148, 150. In some embodiments, to provide an effective tissue seal, an appropriate gap distance of about 0.001 inches to about 0.010 inches and, desirably, between about 0.002 and about 0.005 inches may be provided.

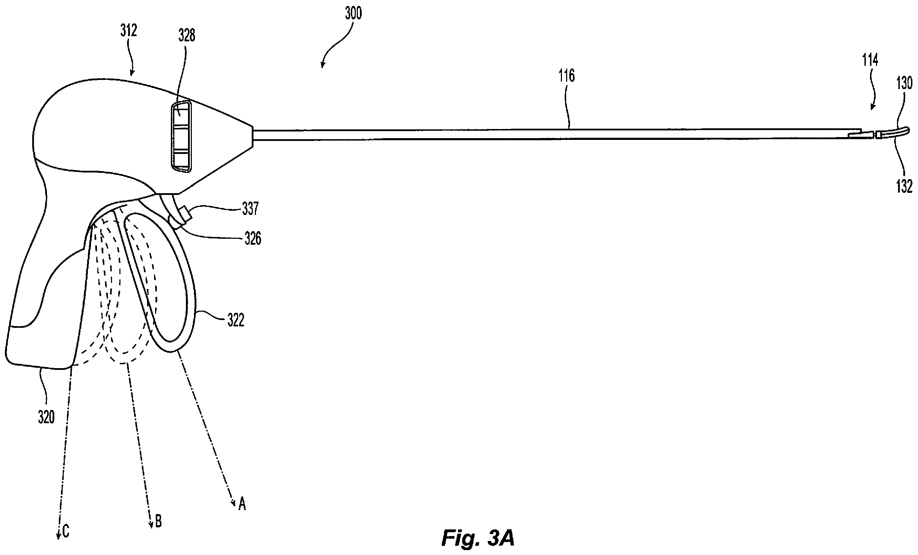

Alternatively, and as detailed below with respect to FIGS. 3A-3D of the present disclosure, the sealing plates 148 and 150 and/or the end effector 114 may be configured for delivering monopolar energy to the tissue. In a monopolar configuration, one or both sealing plates 148 and 150 deliver electrosurgical energy from an active terminal, e.g. (+), while a return pad (not shown) is placed generally on a patient and provides a return path to the opposite terminal, e.g. (-), of the generator 141.

Disposing the switch 137 in line with the actuation of the handle 122 relative to handle 120 allows a user to progressively move through the sealing process by simply actuating the handle 122. In other words, in order to provide a more progressive approach to sealing vessels, in-line activation of switch 137 via actuation of handle 122 through the handle's 122 range of motion initially actuates the jaw members 130, 132 to grasp and compress tissue under the appropriate closure pressures for sealing vessels. Continued actuation of the handle 122 subsequently activates switch 137 for supplying energy to the jaw members 130, 132 for sealing the tissue disposed therebetween. One or more safety features (not shown) may additionally be employed to ensure the jaw members 130, 132 are disposed in the closed or approximated position prior to activation of switch 137, e.g., sensors, internal switches, etc.

As such, and unless a mechanical or electrical override feature (not shown) is included with forceps 100, the jaw members 130, 132 must be disposed in the closed or approximated position prior to energy activation.

FIGS. 3A-3D disclose various embodiments of the presently-disclosed electrosurgical forceps 300, 400, 500, and 600, respectively. Forceps 300 of FIG. 3A includes a housing 312 that supports various actuators thereon for remotely controlling an end effector 114 through an elongated shaft 116. For the purposes herein, all of the embodiments of the forceps 300, 400, 500, and 600 are configured to operably engage shaft 116 and include end effector 114 having jaw members 130, 132 disposed on a distal end thereof.

To mechanically control the end effector 114, the housing 312 supports a stationary handle 320, a movable handle 322, a trigger 326 and a rotation knob 328. The movable handle 322 is operable to move the end effector 114 between the open and closed positions. The trigger 326 is operable to extend and retract a knife blade 156 (see FIG. 2B) through the end effector 114 when the end effector 114 is in the closed configuration. The rotation knob 328 serves to rotate the elongated shaft 116 and the end effector 114 about a longitudinal axis A-A (See FIG. 2A) extending through the forceps 300.

To electrically control the end effector 114, the trigger 326 supports a depressible switch 337 thereon, which is operable by the user to initiate and terminate the delivery of electrosurgical energy to the end effector 114. More specifically, the depressible switch 337 is mechanically coupled to the trigger 336 or is integrally associated with the trigger 336 and is engageable by a user's forefinger. The switch 337 is in electrical communication with generator 141 or a battery (not shown) supported within the housing 312. The generator 141 may include any of the generators described above. In embodiments, the switch 337 may be coupled to a combination Ligasure^{™}, bipolar and monopolar generator 141 (or multiple individual generators) such that, the jaw members 130, 132 may be configured to deliver Ligasure^{™}, bipolar or monopolar energy upon activation of switch 337.

One or more mechanical or electrical features may be employed on or within the housing 312 to control the energy modality from the generator 141. For example, the energy modality of the switch 337 may be dependent on the position of the handle 322 relative to handle 320 (or jaw member 130 relative to jaw member 132). When the handle 322 is disposed in an open or spaced configuration (See position "A" on FIG. 3A) relative to handle 320 (e.g., jaw members 130, 132 are open) and the user activates switch 337, monopolar energy is delivered to one of the jaw members, e.g., jaw member 132, for spot coagulation, blanching or open dissection. If the handle 322 is disposed is a partially closed position (See position "B" on FIG. 3A) but not fully compressed under appropriate closure forces for vessel sealing, the switch 337 may be configured to deliver energy to both jaw members 130, 132 for bipolar coagulation or blending.

When the handle 322 is disposed is a fully closed position (See position "C" on FIG. 3A) and fully compressed to generate the appropriate closure forces for vessel sealing, the switch 337 may be configured to deliver energy according to a specific algorithm, e.g., the Ligasure^{™} energy delivery algorithm, to both jaw members 130, 132 for tissue or vessel sealing (i.e., energy delivery in accordance with an algorithm tailored to produce a tissue seal). In these instances, the position of handle 322 within the housing 312, the drive shaft (not shown) or the jaw members 130, 132 may determine the energy modality either mechanically (e.g., levers, position switches, gears, etc.) or electrically (e.g., one or more sensors). Various safety features or feedback mechanisms may be employed to ensure user coordination with the energy modality, tactile sensors, audible prompts, etc.

In order not to activate the knife 156 when depressing the switch 337 disposed on trigger 326, the force (measured in Newtons (N)) required for depressing and activating the switch 337 is lower than the force required to advance the knife 156. For example, the switch 337 may be configured to activate upon a 6N force whereas the knife 156 begins to advance via actuation of trigger 326 with a 12N force. With certain energy modalities, e.g., monopolar, the amount of force needed to activate the switch 337 versus advance the knife 156 may be irrelevant as the knife 156 may be impeded from advancing when the jaw members 130, 132 are open.

As can be appreciated, providing the switch 337 on the trigger 326 facilitates actuation and is more ergonomically friendly. The user does not have to reposition his/her hand or thumb (or take his/her hand off of the forceps 300) to activate the switch 337.

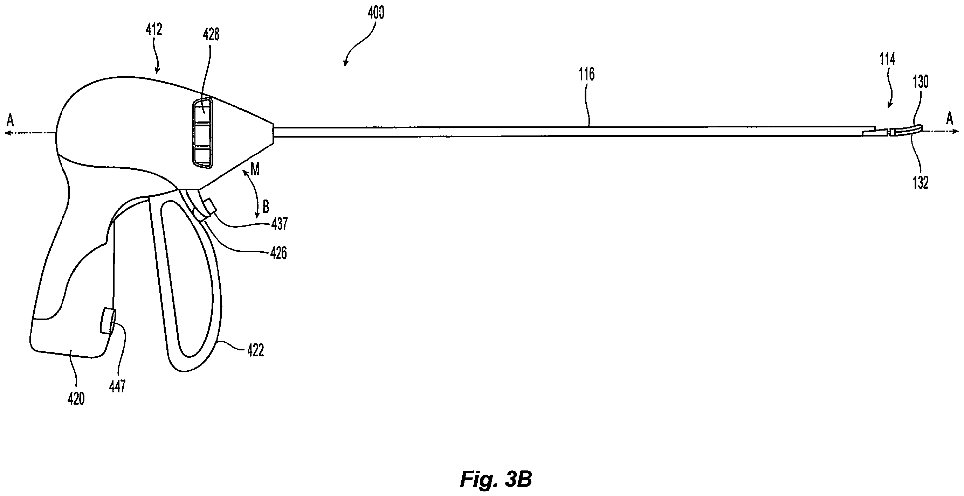

FIG. 3B shows another embodiment of an electrosurgical forceps in accordance with the present disclosure generally identified as forceps 400. Forceps 400 includes many of the same features as forceps 10, 100 and 300 and, as such, only those features that are different will be described in detail. Generally, forceps 400 includes a housing 412 that supports a stationary handle 420, a movable handle 422, a trigger 426 and a rotation knob 428. The movable handle 422 is operable to move the end effector 114 between the open and closed positions. The trigger 426 is operable to extend and retract the knife blade 156 (see FIG. 2B) through the end effector 114 when the end effector 114 is in the closed configuration. The rotation knob 428 rotates the elongated shaft 116 and the end effector 114.

Much like forceps 300, the trigger 426 supports a depressible switch 437 thereon, which is operable by the user to initiate and terminate the delivery of electrosurgical energy to the end effector 114. The switch 437 is in electrical communication with generator 141 or a battery (not shown) supported within the housing 412. Similar to switch 337, switch 437 may be coupled to a combination Ligasure^{™}, bipolar and monopolar generator 141 (or multiple individual generators) such that, the jaw members 130, 132 may be configured to deliver Ligasure^{™}, bipolar or monopolar energy upon activation of switch 437.

Moreover, much like above, one or more mechanical or electrical features may be employed on or within the housing 412 to control the energy modality from the generator 141. For example, the energy modality of the switch 437 may be dependent on the position (e.g., position "A", "B", and "C" of FIG. 3A) of the handle 422 relative to handle 420 (or jaw member 130 relative to jaw member 132). When the handle 422 is disposed in an open position relative to handle 420, monopolar energy is delivered upon activation of switch 437. When the handle 422 is disposed in a partially closed position relative to handle 420, bipolar energy is delivered upon activation of switch 437. When the handle 322 is disposed in fully closed position for vessel sealing, the switch 437 may be configured to deliver Ligasure^{™} energy to both jaw members 130, 132 for tissue or vessel sealing.

Similar to switch 337, the force required for depressing and activating the switch 437 is lower than the force required to advance the knife 156. Providing the switch 437 on the trigger 426 facilitates actuation and is more ergonomically friendly as the user does not have to reposition his/her hand or thumb to activate the switch 437.

Forceps 400 also includes an auxiliary switch 447 disposed on handle 420. Switch 447 is included as an in-line activation switch similar to switch 137 of FIG. 2. More particularly, upon fully compressing the handle 422 relative to handle 420, switch 447 is activated. As can be appreciated, the forceps 400 may be configured such that a user has the option of using either switch 437 or 447 to activate the electrosurgical energy from generator 141. Switch 437 may be configured as a multi-modality energy switch as discussed above with respect to switch 337. Alternatively, switch 447 may be configured to only deliver energy according to a Ligasure^{™} algorithm to seal tissue. Moreover, switch 437 may be a dual-modality switch (e.g., monopolar and bipolar) while switch 447 is a single modality energy switch (e.g., Ligasure^{™}). In these instances, various electrical or mechanical features (not shown) may need to be employed to limit activation of either or both switches 437, 447 depending upon the position of the handle 422 or jaw members 130, 132.

FIG. 3B also shows another embodiment of the switch 437 of the electrosurgical forceps 400 wherein the switch 437 disposed on the trigger 426 is able to move transversally relative to the longitudinal axis A-A defined through the shaft. Movement of the switch 437 in a first direction "M" provides a monopolar energy modality to one or both jaw members 130, 132. Movement of the switch 437 in a second, opposite, direction "B" provides a bipolar energy modality to jaw members 130, 132. Switch 437 may work in conjunction with auxiliary switch 447 as described above.

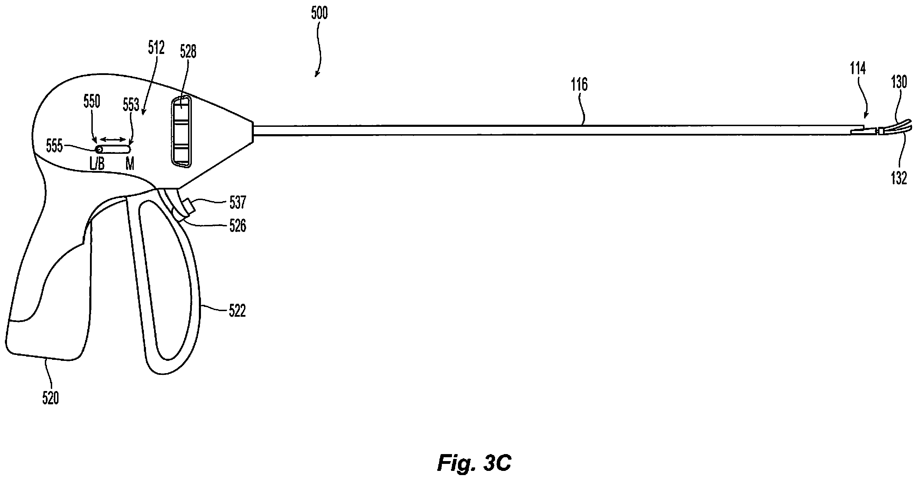

FIG. 3C shows another embodiment of an electrosurgical forceps in accordance with the present disclosure generally identified as forceps 500. Forceps 500 includes many of the same features as forceps 10, 100, 300 and 400 and, as such, only those features that are different will be described in detail. Generally, forceps 500 includes a housing 512 that supports a stationary handle 520, a movable handle 522, a trigger 526 and a rotation knob 528. The movable handle 522 is operable to move the end effector 114 between the open and closed positions. The trigger 526 is operable to extend and retract the knife blade 156 (see FIG. 2B) through the end effector 114 when the end effector 114 is in the closed configuration. The rotation knob 528 rotates the elongated shaft 116 and the end effector 114.

Switch 537 operates in a similar fashion to switches 337 and 437 described above. As such, only the differences will be described herein. Similar to switch 337, switch 537 is in electrical communication with generator 141 or a battery (not shown) supported within the housing 512 and may be coupled to any of the generators 141 described above to deliver Ligasure^{™}, bipolar or monopolar energy upon activation thereof.

Forceps 500 also includes an energy selector 550 disposed on the housing 512. Energy selector 550 includes a channel 553 defined within housing 512 that supports a toggle 555. Toggle 555 is moveable between a first position "L/B" wherein activation of switch 537 will deliver bipolar energy to jaw members 130, 132 or energy according to a Ligasure^{™} algorithm to seal tissue upon activation of switch 537 and a second position "M" wherein activation of switch 537 will deliver monopolar energy to one of the jaw members, e.g., jaw member 132. The energy modality when the toggle 555 is disposed in the first position "L/B" may be determinative on the position of the handle 522 as described above with respect to forceps 300.

Toggle 555 allows forceps 500 to be bifunctional; in a first position used as a monopolar forceps upon activation of switch 537, and in a second position used as a bipolar forceps and/or a Ligasure^{™} forceps for sealing tissue upon activation of switch 537. Isolating the modality of the forceps 500 by moving the toggle 555 between modes "L/B" and "M" and locating the switch 537 on the trigger 526 greatly enhances the ergonomics and functionality of the forceps 500.

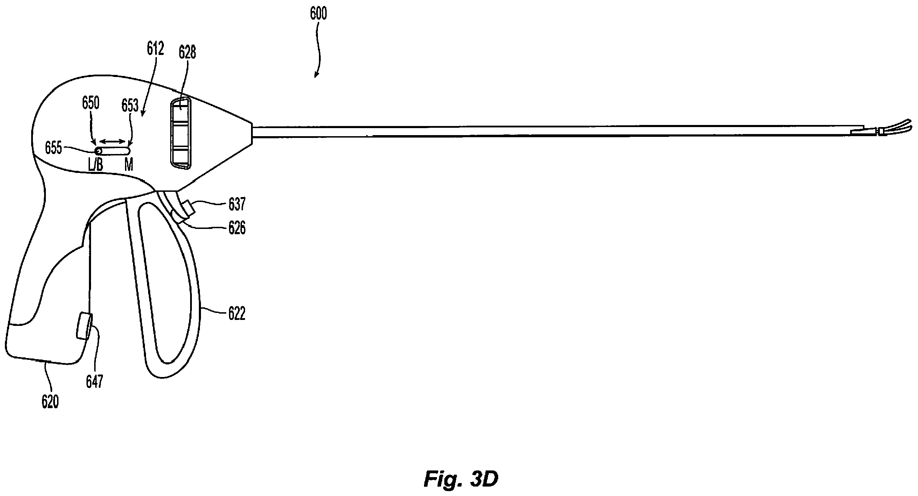

FIG. 3D shows another embodiment of an electrosurgical forceps in accordance with the present disclosure generally identified as forceps 600. Forceps 600 includes many of the same features as forceps 10, 100, 300, 400 and 500 and, as such, only those features that are different will be described in detail. Forceps 600 is essentially a combination of forceps 400 described with respect to FIG. 3B and forceps 500 described with respect to FIG. 3C.

Generally, forceps 600 includes a housing 612 that supports a stationary handle 620, a movable handle 622, a trigger 626 and a rotation knob 628. The movable handle 622 is operable to move the end effector 114 between the open and closed positions. The trigger 626 is operable to extend and retract the knife blade 156 (see FIG. 2B) through the end effector 114 when the end effector 114 is in the closed configuration. The rotation knob 628 rotates the elongated shaft 116 and the end effector 114.

An auxiliary switch 647 disposed on handle 620 acts as an in-line activation switch similar to switch 137 of FIG. 2. Upon fully compressing the handle 622 relative to handle 620, switch 647 is activated. Forceps 600 may be configured such that a user has the option of using either switch 637 or 647 to activate the electrosurgical energy from generator 141. Switch 637 may be configured as a multi-modality energy switch as discussed above with respect to switch 337. Switch 647 may also be configured to only deliver energy according to a Ligasure^{™} algorithm to seal tissue or switch 547 may be a dual-modality switch (e.g., monopolar and bipolar) while switch 647 is a single modality energy switch (e.g., Ligasure^{™}). In these instances, various electrical or mechanical features (not shown) may need to be employed to limit activation of either or both switches 637, 647 depending upon the position of the handle 622 or jaw members 130, 132.

Forceps 600 also includes an energy selector 650 disposed on the housing 612. Much like forceps 500 discussed above, energy selector 650 includes a channel 653 defined within housing 612 that supports a toggle 655. Toggle 655 is moveable between a first position "L/B" wherein activation of switch 637 will deliver bipolar energy to jaw members 130, 132 or energy according to a Ligasure^{™} algorithm to seal tissue upon activation of switch 637 and a second position "M" wherein activation of switch 637 will deliver monopolar energy to one of the jaw members, e.g., jaw member 132. The energy modality when the toggle 655 is disposed in the first position "L/B" may be determinative on the position of the handle 622 as described above with respect to forceps 300.

Toggle 655 allows forceps 600 to be bifunctional; in a first position used as a monopolar forceps upon activation of switch 637, and in a second position used as a bipolar forceps and/or a Ligasure^{™} forceps for sealing tissue upon activation of switch 637. Isolating the modality of the forceps 600 by moving the toggle 655 between modes "L/B" and "M" and locating the switch 637 on the trigger 626 greatly enhances the ergonomics and functionality of the forceps.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs:-
1. An endoscopic surgical forceps, comprising:
   a housing including an elongated shaft having a distal portion extending therefrom and a proximal portion coupled to the housing, the elongated shaft having a longitudinal axis defined therethrough;
   an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the first and second jaw members cooperate to grasp tissue therebetween;
   a first handle pivotably coupled to the housing and movable relative to a second handle disposed on the housing, the first handle operably coupled to the at least one jaw member and configured to actuate the at least one jaw member between the open and closed positions upon movement thereof relative to the second handle;
   a trigger operably coupled to the housing and configured to deploy a knife between the first and second jaw members upon actuation thereof; and
   an energy activation switch adapted to couple to an electrosurgical energy source such that activation thereof supplies energy to at least one of the first and second jaw members, the energy activation switch disposed on the trigger.
2. The endoscopic surgical forceps according to paragraph 1, wherein actuation of the trigger requires a first force and activation of the energy activation switch requires a second force, the first force being greater than the second force.
3. The endoscopic surgical forceps according to paragraph 1, wherein the energy activation switch is adapted to connect to an electrosurgical energy source capable of supplying multiple energy modalities to the jaw members upon activation thereof and wherein the energy modality of the energy activation switch is dependent on the position of the first handle relative to the second handle.
4. The endoscopic surgical forceps according to paragraph 3, wherein when the first handle is disposed in an open position, the energy activation switch, when activated, provides monopolar energy from the electrosurgical energy source to at least one jaw member.
5. The endoscopic surgical forceps according to paragraph 3, wherein when the first handle is disposed in a partially closed position, the energy activation switch, when activated, provides bipolar energy from the electrosurgical energy source to the first and second jaw members.
6. The endoscopic surgical forceps according to paragraph 3, wherein when the first handle is disposed in a fully closed position and is generating a closure pressure within the range of about 3kg/cm² to about 16 kg/cm² between the first and second jaw members, the energy activation switch, when activated, provides energy from the electrosurgical energy source according to an algorithm for sealing tissue between the first and second jaw members.
7. The endoscopic surgical forceps according to paragraph 1, further comprising an auxiliary energy activation switch disposed on the second handle, the auxiliary energy activation switch, upon activation, also configured to provide energy from the electrosurgical energy source according to an algorithm for sealing tissue between the first and second jaw members.
8. The endoscopic surgical forceps according to paragraph 7, wherein the auxiliary energy activation switch is activatable by the first handle moving relative to the second handle from an open position to a fully closed position wherein the first handle generates a closure pressure within the range of about 3kg/cm² to about 16 kg/cm² between the first and second jaw members.
9. The endoscopic surgical forceps according to paragraph 8, wherein the energy activation switch, when activated, provides monopolar or bipolar energy to the first and second jaw members depending upon the position of the first handle relative to the second handle.
10. An endoscopic surgical forceps, comprising:
   a housing including an elongated shaft having a distal portion extending therefrom and a proximal portion coupled to the housing, the elongated shaft having a longitudinal axis defined therethrough;
   an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the jaw members cooperate to grasp tissue therebetween;
   a trigger operably coupled to the housing and configured to deploy a knife between the first and second jaw members upon actuation thereof; and
   an energy activation switch adapted to couple to an electrosurgical energy source such that activation thereof supplies energy to at least one of the first and second jaw members, the energy activation switch disposed on the trigger.
11. The endoscopic surgical forceps according to paragraph 10, wherein the energy activation switch is adapted to connect to an electrosurgical energy source capable of supplying multiple energy modalities to the first and second jaw members upon activation of the energy activation switch and wherein the energy modality of the energy activation switch is dependent the actuation direction of the energy activation switch relative to the longitudinal axis.
12. An endoscopic surgical forceps, comprising:
   a housing including an elongated shaft having a distal portion extending therefrom and a proximal portion coupled to the housing, the elongated shaft having a longitudinal axis defined therethrough;
   an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the jaw members cooperate to grasp tissue therebetween;
   a trigger operably coupled to the housing and configured to deploy a knife between the first and second jaw members upon actuation thereof;
   an energy activation switch adapted to couple to an electrosurgical energy source such that activation thereof supplies energy to at least one of the first and second jaw members, the energy activation switch disposed on the trigger; and
   a toggle disposed on the housing and configured to change an energy modality of the energy activation switch between at least two energy modalities.
13. The endoscopic surgical forceps according to paragraph 12, wherein the toggle is moveable between a first position enabling the supply monopolar energy from the electrosurgical energy source to at least one of the jaw members upon activation of the energy activation switch and a second position enabling the supply of bipolar energy from the electrosurgical energy source to the first and second jaw members upon activation of the energy activation switch.
14. The endoscopic surgical forceps according to paragraph 12, further comprising an auxiliary energy activation switch disposed on the second handle, the auxiliary energy activation switch, upon activation, also configured to provide energy from the electrosurgical energy source according to an algorithm for sealing tissue between the first and second jaw members.
15. The endoscopic surgical forceps according to paragraph 14, wherein the auxiliary energy activation switch is activatable by the first handle moving relative to the second handle from an open position to a fully closed position wherein the first handle generates a closure pressure within the range of about 3kg/cm² to about 16 kg/cm² between the first and second jaw members.
16. The endoscopic surgical forceps according to paragraph 15, wherein the energy activation switch, when activated, provides monopolar or bipolar energy to the first and second jaw members depending upon the position of the first handle relative to the second handle.

## Claims

1. An endoscopic surgical forceps, comprising:
a housing including an elongated shaft having a distal portion extending therefrom and a proximal portion coupled to the housing, the elongated shaft having a longitudinal axis defined therethrough;
an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the first and second jaw members cooperate to grasp tissue therebetween;
a first handle pivotably coupled to the housing and movable relative to a second handle disposed on the housing, the first handle operably coupled to the at least one jaw member and configured to actuate the at least one jaw member between the open and closed positions upon movement thereof relative to the second handle;
a trigger operably coupled to the housing and configured to deploy a knife between the first and second jaw members upon actuation thereof; and
an energy activation switch adapted to couple to an electrosurgical energy source such that activation thereof supplies energy to at least one of the first and second jaw members, the energy activation switch disposed on the trigger.

2. The endoscopic surgical forceps according to claim 1, wherein actuation of the trigger requires a first force and activation of the energy activation switch requires a second force, the first force being greater than the second force.

3. The endoscopic surgical forceps according to claim 1 or 2, wherein the energy activation switch is adapted to connect to an electrosurgical energy source capable of supplying multiple energy modalities to the jaw members upon activation thereof and wherein the energy modality of the energy activation switch is dependent on the position of the first handle relative to the second handle.

4. The endoscopic surgical forceps according to claim 3, wherein when the first handle is disposed in an open position, the energy activation switch, when activated, provides monopolar energy from the electrosurgical energy source to at least one jaw member.

5. The endoscopic surgical forceps according to claim 3, wherein when the first handle is disposed in a partially closed position, the energy activation switch, when activated, provides bipolar energy from the electrosurgical energy source to the first and second jaw members.

6. The endoscopic surgical forceps according to claim 3, wherein when the first handle is disposed in a fully closed position and is generating a closure pressure within the range of about 3kg/cm² to about 16 kg/cm² between the first and second jaw members, the energy activation switch, when activated, provides energy from the electrosurgical energy source according to an algorithm for sealing tissue between the first and second jaw members.

7. The endoscopic surgical forceps according to any preceding claim, further comprising an auxiliary energy activation switch disposed on the second handle, the auxiliary energy activation switch, upon activation, also configured to provide energy from the electrosurgical energy source according to an algorithm for sealing tissue between the first and second jaw members.

8. The endoscopic surgical forceps according to claim 7, wherein the auxiliary energy activation switch is activatable by the first handle moving relative to the second handle from an open position to a fully closed position wherein the first handle generates a closure pressure within the range of about 3kg/cm² to about 16 kg/cm² between the first and second jaw members.

9. The endoscopic surgical forceps according to claim 8, wherein the energy activation switch, when activated, provides monopolar or bipolar energy to the first and second jaw members depending upon the position of the first handle relative to the second handle.

10. An endoscopic surgical forceps, comprising:
a housing including an elongated shaft having a distal portion extending therefrom and a proximal portion coupled to the housing, the elongated shaft having a longitudinal axis defined therethrough;
an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the jaw members cooperate to grasp tissue therebetween;
a trigger operably coupled to the housing and configured to deploy a knife between the first and second jaw members upon actuation thereof; and
an energy activation switch adapted to couple to an electrosurgical energy source such that activation thereof supplies energy to at least one of the first and second jaw members, the energy activation switch disposed on the trigger.

11. The endoscopic surgical forceps according to claim 10, wherein the energy activation switch is adapted to connect to an electrosurgical energy source capable of supplying multiple energy modalities to the first and second jaw members upon activation of the energy activation switch and wherein the energy modality of the energy activation switch is dependent the actuation direction of the energy activation switch relative to the longitudinal axis.

12. An endoscopic surgical forceps, comprising:
a housing including an elongated shaft having a distal portion extending therefrom and a proximal portion coupled to the housing, the elongated shaft having a longitudinal axis defined therethrough;
an end effector assembly operably coupled to a distal end of the elongated shaft, the end effector including first and second jaw members, at least one of the jaw members movable relative to the other jaw member between an open position wherein the at least one jaw member is spaced relative to the other jaw member and a closed position wherein the jaw members cooperate to grasp tissue therebetween;
a trigger operably coupled to the housing and configured to deploy a knife between the first and second jaw members upon actuation thereof;
an energy activation switch adapted to couple to an electrosurgical energy source such that activation thereof supplies energy to at least one of the first and second jaw members, the energy activation switch disposed on the trigger; and
a toggle disposed on the housing and configured to change an energy modality of the energy activation switch between at least two energy modalities.

13. The endoscopic surgical forceps according to claim 12, wherein the toggle is moveable between a first position enabling the supply monopolar energy from the electrosurgical energy source to at least one of the jaw members upon activation of the energy activation switch and a second position enabling the supply of bipolar energy from the electrosurgical energy source to the first and second jaw members upon activation of the energy activation switch.

14. The endoscopic surgical forceps according to claim 12 or 13, further comprising an auxiliary energy activation switch disposed on the second handle, the auxiliary energy activation switch, upon activation, also configured to provide energy from the electrosurgical energy source according to an algorithm for sealing tissue between the first and second jaw members.

15. The endoscopic surgical forceps according to claim 14, wherein the auxiliary energy activation switch is activatable by the first handle moving relative to the second handle from an open position to a fully closed position wherein the first handle generates a closure pressure within the range of about 3kg/cm² to about 16 kg/cm² between the first and second jaw members, preferably wherein the energy activation switch, when activated, provides monopolar or bipolar energy to the first and second jaw members depending upon the position of the first handle relative to the second handle.
